Europäisches Patentamt

European Patent Office

Office européen des brevets

(11) Veröffentlichungsnummer: **0 072 919**
**B1**

(12) **EUROPÄISCHE PATENTSCHRIFT**

(45) Veröffentlichungstag der Patentschrift:
08.01.86

(51) Int. Cl.⁴: **C 07 D 233/92**

(21) Anmeldenummer: **82106392.2**

(22) Anmeldetag: **16.07.82**

(54) Verfahren zur Gewinnung von 1,2-Dimethyl-5-nitroimidazol von hoher Reinheit.

(30) Priorität: **13.08.81 DE 3132025**

(43) Veröffentlichungstag der Anmeldung:
**02.03.83 Patentblatt 83/9**

(45) Bekanntmachung des Hinweises auf die Patenterteilung:
**08.01.86 Patentblatt 86/2**

(84) Benannte Vertragsstaaten:
**DE FR GB IT**

(56) Entgegenhaltungen:
**EP - A - 0 043 922**
**DE - A - 1 795 709**
**DE - A - 2 414 280**
**FR - A - 2 263 241**
**GB - A - 1 418 538**

(73) Patentinhaber: **BASF Aktiengesellschaft,
Carl-Bosch-Strasse 38, D-6700 Ludwigshafen (DE)**

(72) Erfinder: **Dockner, Toni, Dr., Grossgasse 6,
D-6701 Meckenheim (DE)**
Erfinder: **Frank, Anton, Bannwasserstrasse 72,
D-6700 Ludwigshafen (DE)**
Erfinder: **Riewe, Guenter, Kalmitring 17,
D-6701 Dannstadt-Schauernheim (DE)**
Erfinder: **Wetzler, Matthias, Liebermannstrasse 7,
D-6700 Ludwigshafen (DE)**

## Beschreibung

Die Erfindung betrifft ein neues Verfahren zur Gewinnung von 1,2-Dimethyl-5-nitroimidazol von hoher Reinheit.

1,2-Dimethyl-5-nitroimidazol (kurz als Dimetridazol bezeichnet) lässt sich durch Umsetzung von 2-Methyl-4(5)- nitroimidazol mit Dimethylsulfat in einem Lösungsmittel herstellen. Nach dem in Arzneimittelforschung, Band 16 (1966), S. 28, beschriebenen Verfahren wird Dimetridazol mit einer Ausbeute von 54% d.Th. erhalten, wenn man 2-Methyl-4(5)-nitroimidazol in Toluol mit Dimethylsulfat umsetzt. Man erhält dabei durch Zugabe von Ammoniak zum Reaktionsgemisch ein Rohprodukt, das durch Behandeln mit Chloroform und Umkristallisieren aus Ethylacetat gereinigt werden muss. Ein vorteilhafteres Verfahren wird in der DE-OS 24 14 280 beschrieben, bei dem man Ameisensäure als Lösungsmittel verwendet, das nach der Umsetzung des 2-Methyl-4(5)-nitroimidazols mit Dimethylsulfat abdestilliert wird. Der Rückstand wird in Wasser gelöst, mit Ammoniakwasser auf einen pH von 1 bis 3 eingestellt, wobei unumgesetztes 2-Methyl-4(5)-nitroimidazol ausfällt. Nach Abkühlung der sauren Lösung und Abtrennung des Ausgangsstoffes wird das Filtrat z.B. mit Ammoniak auf einen pH von 8 bis 14 eingestellt und das Dimetridazol abgetrennt. Nach diesem Verfahren erhält man ein Dimetridazol mit einer Reinheit von 97 bis 98,5% und einem Anteil an unumgesetztem 2-Methyl-4(5)-nitroimidazol von 1,0 bis 2,8 Gew.-%.

Dimetridazol wird in der Tiermedizin bei der Behandlung von Histomoniasis und von Schweinedysenterie eingesetzt. Für diesen Verwendungszweck wird eine hohe Reinheit gefordert. So wird im British Pharmacopoeia 1977 eine Reinheit von über 99,5% und ein Gehalt an 2-Methyl-4(5)-nitroimidazol von unter 0,5 Gew.-% verlangt. Da Dimetridazol von solcher Reinheit bei dem genannten Verfahren nicht anfällt und es somit erforderlich ist, das Dimetridazol durch mehrmalige Umkristallisation zu reinigen, war nach einem Verfahren zu suchen, das es gestattet 1,2-Dimethyl-5-nitroimidazol auf einfacherem Wege in der geforderten hohen Reinheit zu gewinnen.

Es wurde nun gefunden, dass man 1,2-Dimethyl-5-nitroimidazol von hoher Reinheit aus einer wässrigen sauren Lösung, die 1,2-Dimethyl-5-nitroimidazol enthält, besonders vorteilhaft dadurch gewinnen kann, dass man die wässrige saure Lösung bei 70 bis 105 °C auf einen pH-Wert von 4 bis 6,5 einstellt, durch Abkühlung auf unter 30 °C zur Kristallisation bringt und das auskristallisierte 1,2-Dimethyl-5-nitroimidazol abtrennt.

Nach dem neuen Verfahren isoliert man das reine Dimetridazol aus seinen wässrigen sauren Lösungen, die man z.B. nach dem in der DE-OS 24 14 280 beschriebenen Verfahren durch Umsetzung von 2-Methyl-4(5)-nitroimidazol mit Dimethylsulfat in Ameisensäure bei Temperaturen von 20 bis 110 °C, Abdestillieren der Ameisensäure, Zugabe von Wasser, Einstellen der sauren Reaktionslösung auf einen pH-Wert von 1 bis 3 und Abtrennung des nicht umgesetzten Ausgangsstoffes aus der abgekühlten wässrigen Lösung erhält. Diese wässrigen Lösungen, aus denen man nach dem erfindungsgemässen Verfahren das hochreine Dimetridazol gewinnt, enthalten z.B. 0,1 bis 15 Gew.-% 1,2-Dimethyl-5-nitroimidazol, 0,5 bis 1 Gew.-% 2-Methyl-4-(5)-nitroimidazol, 1 bis 5 Gew.-% Ameisensäure, 0,2 bis 0,3 Gew.-% 1,2-Dimethyl-4-nitromidazol und 0,05 bis 0,1 Gew.-% 1-Methyl-5-nitroimidazol.

Die sauren wässrigen Ausgangslösungen werden bei Temperaturen von 70 bis 105 °C, vorzugsweise 85 bis 90 °C, durch Zugabe einer Base auf einen pH-Wert von 4 bis 6,5 eingestellt. Als Basen verwendet man z.B. Natriumcarbonat, Natriumhydroxid, Kaliumhydroxid oder Ammoniak, vorteilhaft in Form der wässrigen Lösungen, insbesondere wässriges 5- bis 30%iges Ammoniak. Dann kühlt man auf unter 30 °C ab, vorzugsweise auf Temperaturen von 0 bis 25 °C, wodurch man die Lösung zur Kristallisation bringt. Das auskristallisierte 1,2-Dimethyl-5-nitroimidazol wird dann auf an sich übliche Weise abgetrennt, z.B. durch Filtration oder mit Hilfe einer Zentrifuge, wobei man die Kristalle vor ihrer Trocknung gegebenenfalls mit wässrigem Ammoniak wäscht. Man erhält das Dimetridazol hierbei in einer Reinheit von über 99% und mit einem Gehalt an 2-Methyl-4(5)-nitroimidazol von weniger als 0,5 Gewichtsprozent. Das Verfahrensprodukt wird in Kristallen der Grössen 30 bis 300 μ erhalten, die auch bei längerem Lagern nicht verbacken. Das nach dem Verfahren der DE-OS 24 14 280 hergestellte Dimetridazol hat demgegenüber eine Kristallgrösse von 4 bis 30 μ und neigt dazu, bereits nach kurzer Lagerung irreversibel zu verbacken. Das erfindungsgemäss gewonnene Dimetridazol weist als weiteren Vorteil im Vergleich zu den bekannten Verfahrensprodukten ein höheres Schüttgewicht auf.

Beispiel 1

In einen 2000 l – Emailrührbehälter werden 560 kg 2-Methyl-4(5)-nitroimidazol und 800 kg Ameisensäure eingefüllt. Unter Vakuum (−900 mb) wird unter Rühren aufgeheizt, bis bei 54 bis 56 °C die Ameisensäure schwach am Rückfluss kocht. Dann lässt man aus einer Vorlage 421 l = 560 kg Dimethylsulfat innerhalb von 2 Stunden zulaufen. Das Reaktionsgemisch wird anschliessend noch 30 Minuten unter Rückfluss gerührt. Bei stufenweiser Absenkung des Druckes und Beheizung mit 1,5 bar Dampf destilliert man nun innerhalb von 8 Stunden die Ameisensäure ab. Dann wird bei einer Sumpftemperatur von 68 °C und einem Druck von ∼50 mbar die Beheizung abgestellt.

Das Reaktionsgemisch wird durch Zugabe von 600 l Wasser verdünnt, dann wird der Rührbehälter entspannt. Die saure wässrige Lösung wird in einen 2 m³-V2A-Rührbehälter gepumpt und durch Zugabe von 25%igem wässrigem Ammoniak auf pH 1,25 eingestellt. Dabei fällt das nichtumgesetzte 2-Methyl-4(5)-nitroimidazol aus, welches über eine Pendelzentrifuge abgetrennt wird. Man erhält 250 kg feuchtes 2-Methyl-4(5)-nitromidazol. Die Mutterlauge wird mit Kohle geklärt, filtriert und in einen weiteren 2,5 m³-V2A-Rührbehälter gepumpt.

Die wässrige saure Mutterlauge wird unter Rühren mit 1,5 bar Dampf auf 70 °C aufgeheizt. Dann wird die Heizung abgestellt. Innerhalb von 20 Minuten gibt man 420 bis 440 l 25%iges wässriges Ammoniak so hinzu, dass der pH-Wert 4,2 erreicht wird. Dabei steigt die Temperatur auf 92 bis 93 °C an. Danach wird mit Kühlwasser unter Rühren so abgekühlt, dass die Temperatur innerhalb 1 Stunde auf 80 °C und innerhalb weiterer 3 Stunden auf 25 °C abfällt, wobei die Mutterlauge zu kristallisieren beginnt.

Das auskristallisierte Dimetridazol wird auf einer Zentrifuge abgetrennt, mit 300 l 25%igem wässrigem Ammoniak gewaschen und in einem Trockner getrocknet. Man erhält 312 kg, 1,2-Dimethyl-5-nitroimidazol, das sind 85% der Theorie, bezogen auf 2-Methyl-4(5)-nitroimidazol. Das 1,2-Dimethyl-5-nitroimidazol hat eine Reinheit von 99,5%. Das Produkt enthält 0,2% 2-Methyl-4(5)-nitroimidazol und 0,1% 1,2-Dimethyl-4-nitroimidazol. Es ist gut rieselfähig und verbackt nicht. Seine Korngrössenverteilung ist 30 bis 300 μ (50% > 100 μ).

Beispiel 2 (Vergleichsbeispiel)

Man stellt 1,2-Dimethyl-5-nitroimidazol gemäss Beispiel 1 der DE-OS 24 14 280 folgendermassen her:

600 Teile Ameisensäure (85%ig), 225 Teile 2-Methyl-5-nitroimidazol und 250 Teile Dimethylsulfat werden in einem Rührkessel 4 Stunden unter Rückfluss erhitzt (80 °C). Danach wird die Ameisensäure im Vakuum abdestilliert. Der verbleibende Rückstand wird in 400 Teilen Wasser gelöst, mit Ammoniakwasser auf pH 1,8 abgestumpft und auf 0 bis +5 °C gekühlt. Das unumgesetzte 2-Methyl-5-nitroimidazol fällt aus und wird abgeschleudert. Das Gemisch wird dann mit Ammoniakwasser auf pH 10 eingestellt und bei 10 °C 1,2-Dimethyl-5-nitroimidazol abgetrennt. Man erhält 75 Teile 2-Methyl-5-nitroimidazol und 145 Teile 1,2-Dimethyl-5-nitroimidazol vom Fp. 137 bis 140 °C. Die Ausbeute, bezogen auf umgesetztes 2-Methyl-5-nitroimidazol, beträgt 84% der Theorie. Das 1,2-Dimethyl-5-nitroimidazol hat eine Reinheit von 97,7%. Es enthält 1,3 Gew.-% 2-Methyl-5-nitroimidazol und 0,5% 1,2-Dimethyl-4-nitroimidazol. Beim Lagern ist es nach einer Zeit von 10 Stunden irreversibel verbackt.

Beispiel 3

3 kg einer technisch anfallenden etwa 10%igen wässrigen Lösung von 1,2-Dimethyl-5-nitroimidazol mit einem pH-Wert von 1,7, die als Verunreinigungen u.a. 2-Methyl-4-nitroimidazol, 1-Methyl-5-nitroimidazol und 1,2-Dimethyl-4-nitroimidazol enthält, werden auf 85 °C erwärmt und innerhalb 20 Minuten mit 635 ml 25%igem Ammoniakwasser versetzt, wobei sich ein pH-Wert von 4,8 einstellt. Die Temperatur steigt auf 103 °C. Nach etwa 2 Minuten wird Wasserstrahlvakuum angelegt und ständig verbessert, so dass die Temperatur der Lösung durch Siedekühlung in etwa 1 Stunde auf 12 °C fällt (bei einem Druck von 10 mbar), wobei sie kristallisiert. Die ausgeschiedenen, groben Kristalle werden abgesaugt, mit 600 ml kaltem Ammoniakwasser gewaschen und getrocknet. Man erhält 334 g Dimetridazol vom Schmelzpunkt 135,3 bis 138 °C. Es hat eine Reinheit von 99,67%. Sein Schüttgewicht beträgt 565 bis 764 g/l. Das Produkt enthält nach der HPLC-Analyse 0,18% 2-Methyl-4-nitroimidazol und 0,08% 1-Methyl-5-nitroimidazol.

**Patentansprüche**

1. Verfahren zur Gewinnung von 1,2-Dimethyl-5-nitroimidazol von hoher Reinheit aus einer wässrigen sauren Lösung, die 1,2-Dimethyl-5-nitroimidazol enthält, dadurch gekennzeichnet, dass man die wässrige saure Lösung bei 70 bis 105 °C auf einen pH-Wert von 4 bis 6,5 einstellt, durch Abkühlung auf unter 30 °C zur Kristallisation bringt und das auskristallisierte 1,2-Dimethyl-5-nitroimidazol abtrennt.

2. Verfahren nach Anspruch 1, dadurch gekennzeichnet, dass man die wässrige saure Lösung durch Zugabe einer Base auf einen pH-Wert von 4 bis 6,5 einstellt.

3. Verfahren nach Anspruch 2, dadurch gekennzeichnet, dass man als Base Natriumcarbonat, Natriumhydroxid, Kaliumhydroxid oder Ammoniak verwendet.

**Revendications**

1. Procédé de préparation de 1,2-diméthyl-5-nitroimidazole de haute pureté à partir d'une solution aqueuse acide, qui contient du 1,2-diméthyl-5-nitroimidazole, caractérisé en ce qu'on ajuste la solution aqueuse acide, à 70 à 105 °C, à un pH de 4 à 6,5, en ce qu'on l'amène par refroidissement à moins de 30 °C, et en ce qu'on sépare le 1,2-diméthyl-5-nitroimidazole qui cristallise.

2. Procédé selon la revendication 1, caractérisé en ce qu'on ajuste la solution aqueuse acide par addition d'une base à un pH de 4 à 6,5.

3. Procédé selon la revendication 2, caractérisé en ce qu'on utilise comme base du carbonate de sodium, de l'hydroxyde de sodium, de l'hydroxyde de potassium ou de l'ammoniaque.

**Claims**

1. A process for obtaining 1,2-dimethyl-5-nitroimidazole of high purity from an aqueous acid solution containing 1,2-dimethyl-5-nitroimidazole, wherein the aqueous acid solution is adjusted to a pH of from 4 to 6.5 at 70 to 105 °C and then caused to crystallize by cooling to below 30 °C, and the 1,2-dimethyl-5-nitroimidazole which has crystallized out is separated off.

2. A process as claimed in claim 1, wherein the aqueous acid solution is adjusted to a pH of from 4 to 6.5 by the addition of a base.

3. A process as claimed in claim 2, wherein sodium carbonate, sodium hydroxide, potassium hydroxide or ammonia is used as the base.